# EUROPEAN PATENT SPECIFICATION

(11) **EP 2 120 826 B1**
(45) Date of publication and mention of the grant of the patent: **13.07.2011**
(21) Application number: 08730129.7
(22) Date of filing: 19.02.2008
(51) Int. Cl.: A61F 13/15

(54) **PRINTED WEB**
DRUCKROLLE
BANDE IMPRIMÉE

(30) Priority: 23.02.2007 US 710214; 23.02.2007 US 710215; 23.02.2007 US 710367
(43) Date of publication of application: 25.11.2009
(73) Proprietor: The Procter & Gamble Company, Cincinnati, OH 45202 (US)
(72) Inventor: OETJEN, David, Christopher, West Chester, Ohio 45069 (US); UEMINAMI, Atsushi, Hyogo (JP); WEISMAN, Paul, Thomas, Cincinnati, Ohio 45241 (US)
(74) Representative: Kremer, Véronique Marie Joséphine
(86) International application number: PCT/US2008/054267
(87) International publication number: WO 2008/103650

(56) References cited:
- EP-A- 1 295 711
- EP-A- 1 818 032
- WO-A-2006/009996
- US-A1- 2005 096 614
- US-B1- 6 353 149

## Description

### FIELD OF THE INVENTION

This invention relates to webs useful for topsheets on disposable absorbent products, and more specifically to disposable absorbent products having visible images visible on a topsheet thereof.

### BACKGROUND OF THE INVENTION

Disposable absorbent articles, such as disposable diapers, sanitary napkins, pantiliners, interlabial devices, incontinent devices, training pants, tampons, and the like, are known in the art. Printing on or below the top surface of an absorbent article is known in the art. Printing to create a signal that masks stains, for example, is known. One currently-marketed disposable absorbent article, ALWAYS® brand sanitary napkins, there is printed on one layer underlying the topsheet a color signal that is visible through the topsheet. By printing on a layer below the topsheet, the color signal can be viewed through the topsheet to provide for a perception of depth within the absorbent article. Depth perception of a printed color signal appears to be particularly effective when utilized with a three-dimensional formed film topsheet, as used on the aforementioned ALWAYS® brand sanitary napkin.

US 2005/0096614 A1 discloses a cover layer for a feminine care product having indicia printed thereon.

EP 1 295 711 A1 discloses a color printed liquid permeable laminate structure for use in disposable absorbent articles.

EP 1 818 032 A1 discloses an absorbent article having stain resistant properties.

It would be desirable to gain the demonstrated advantages of printing a color signal on a layer below the topsheet of a disposable absorbent article in an alternative manner that either (1) does not require an underlying layer, or (2) can permit elimination of a layer of material, or (3) utilizes less ink or other printed medium to achieve an acceptable level of visual perception by a user.

### SUMMARY OF THE INVENTION

A web having printed thereon a colorant is disclosed. The web can be a three-dimensional, fluid pervious, polymeric web or a nonwoven web. The web can comprise a pattern of protrusions, a pattern of interconnecting members, the interconnecting members defining apertures. The apertures are defined in a first surface of the web in a first plane of the web, and extend in sidewall portions to a second surface in a second plane of the web. A colorant can be deposited on at least a X portion of the second surface of the web.

### BRIEF DESCRIPTION OF THE DRAWINGS

FIG. 1 is a perspective view of a portion of a three-dimensional formed film suitable for use in the present invention.
FIG. 2 is a perspective view of a three-dimensional formed film suitable for use in the present invention.
FIG. 3 is a perspective view of a three-dimensional formed film suitable for use in the present invention.
FIG. 4 is an enlarged perspective view of a portion of the three-dimensional formed film shown in FIG. 3.
FIG. 5 is a perspective view of a three-dimensional formed film of the present invention.
FIG. 6 is a perspective view of a disposable absorbent article of the present invention.
FIG. 7 is a schematic representation of a flexographic printing process of the present invention.
FIG. 8 is a detail of the printing process shown in FIG. 7.
FIG. 9 is a photomicrograph of a web of the present invention.
FIG. 10 is a photomicrograph of a web of the present invention.
FIG.11 is a schematic cross section of a nonwoven web of the present invention.

### DETAILED DESCRIPTION OF THE INVENTION

A web, and method and apparatus for making a web are disclosed. The web can be beneficially utilized as a topsheet or other component in a disposable absorbent article. The web can be a nonwoven web or a polymeric film, as each are known in the art for use as topsheets in disposable absorbent articles. In one embodiment, the web can be a nonwoven web that is locally deformed, such as: by embossing, to have out-of-plane, i.e., "Z-direction" three-dimensionality. "Z-direction" as used herein has its ordinary meaning as used in the art of nonwoven webs and absorbent articles, and refers to the direction generally orthogonal to the X-Y plane of a generally planar, generally flat web. An embossment or other perturbation made in a generally flat, planar, nonwoven web is considered to be extended in the Z-direction. Z-direction perturbations in a web can be macroscopic, i.e., visible with the unaided eye at a distance of about 30.5cm (12 inches) and as such can effectively increase the bulk of the web. Bulk is a measure that is the inverse of density, i.e., volume per unit mass, and can be increased by increasing the effective thickness of the web without increasing the web's basis weight. The three dimensional protrusions formed by the perturbations can define apertures, and can be uniform in a Z-direction dimensions (i.e, "height") such that the distal ends of the protrusions can be considered to end in a common plane, or surface, as described below with respect to formed films. The web can be a formed film, such as a macroscopically-expanded, three-dimensional, fluid pervious, polymeric web. The description below is in the context of a formed film embodiment, but it is to be understood that in principle, the web, apparatus, and methods disclosed can be adapted to a three-dimensionally formed nonwoven web.

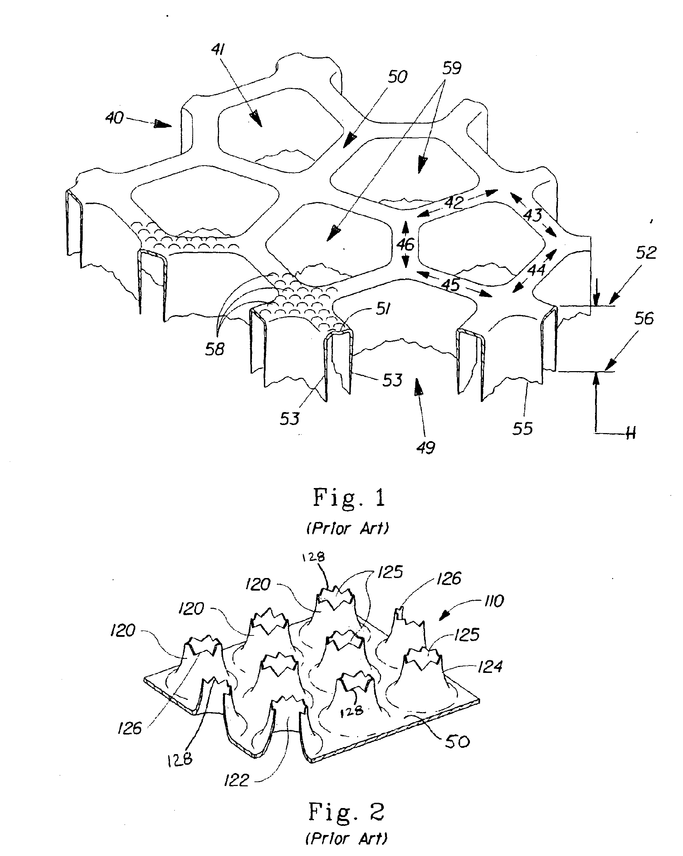

FIG. 1 is an enlarged, partially segmented perspective illustration of a prior art macroscopically-expanded, three-dimensional, fluid pervious polymeric web **40** formed generally in accordance with U.S. Pat. No. 4,342,314 issued to Radel et al. on Aug. 3, 1982. Webs of this type have been found to be highly suitable for use as a topsheet in absorbent articles such as sanitary napkins, pantiliners, interlabial devices, and the like. The fluid pervious web **40** exhibits a plurality of macroscopic surface aberrations that can be apertures, such as primary apertures **41**. Primary apertures **41** are formed by a multiplicity of interconnecting members, such as fiber like elements, e.g., **42, 43, 44, 45** and **46**, that are interconnected to one another to define a continuous first surface **50** of the web **40**. Each fiber like element has a base portion, e.g., base portion **51**, located in plane **52** of first surface **50**. Each base portion has a sidewall portion, e.g., sidewall portion **53**, attached to each longitudinal edge thereof. The sidewall portions extend generally in the direction of a discontinuous second surface **55** of web **40**. The intersecting sidewall portions are interconnected to one another intermediate the first and second surfaces of the web, and terminate substantially concurrently with one another in the plane **56** of the second surface **55**. Planes **52** and **56** can be substantially parallel and separated by a distance termed herein as thickness or caliper **H**. In some embodiments, the base portion **51** may have surface aberrations **58** in accordance with U.S. Patent No. 4,463,045 issued to Ahr et al. on July 31, 1984.

As used herein, the term "macroscopically expanded" refers to the structure of a web formed from a precursor web or film, e.g., a planar web, that has been caused to conform to the surface of a three-dimensional forming structure so that both sides, or surfaces, of the precursor web are permanently altered due to at least partial conformance of the precursor web to the three-dimensional pattern of the forming structure. The three-dimensional structure results in a first surface (e.g., first surface 50) and a second surface (e.g., second surface 55) that are a predetermined distance apart, the predetermined distance being at least twice, three times, five times, or ten times the thickness or the precursor web or film. Such macroscopically-expanded webs are typically caused to conform to the surface of the forming structure by embossing (i.e., when the forming structure exhibits a pattern comprised primarily of male projections), by debossing (i.e., when the forming structure exhibits a pattern comprised primarily of female depressions, or aperture), or by a combination of both.

As used herein, the term "macroscopic" refers to structural features or elements that are readily visible and distinctly discernable to a human having 20/20 vision when the perpendicular distance between the viewer's eye and the web is about 30.5cm (12 inches). Conversely, the term "macroscopic" is utilized to refer to structural features or elements that are not readily visible and distinctly discernable to a human having 20/20 vision when the perpendicular distance between the viewer's eye and the plane of the web is about 30.5 cm (12 inches). In general, as used herein, the primary apertures of a web disclosed herein are macroscopic, and surface aberrations, such as hair-like fibrils as disclosed more fully below are considered microscopic.

The term "planar" as used herein to refers to the overall condition of a precursor web or film when viewed by the naked eye on a macroscopic scale, prior to permanently deforming the web into a three-dimensional formed film. In this context, extruded films prior to post-extrusion processing and films that do not exhibit significant degree of permanent macroscopic three-dimensionality, e.g., deformation out of the plane of the film, would generally be described as planar.

As utilized herein, the term "polymeric web" refers to webs comprised substantially completely of polymeric material, such as polymeric films and nonwovens. The term "polymeric film" refers film polymeric webs, including webs made of polyethylene or polypropylene, and commonly used for topsheets and backsheets of disposable absorbent articles. Polymeric films can be made by means well known in the art, including extrusion, cast, blown, and laminate methods such as co-extrusion. The term "nonwoven" or "nonwoven web" refers to nonwoven polymeric webs made of polymeric fibers, such as polyethylene, propylene, PET, and combinations and blends, including bicomponent fibers, split fibers, and staple fibers. Nonwoven webs can be made by means well known in the art, including meltblown, spunlace, carded, airlaid, spunbond, and the like.

As utilized herein, the term "interconnecting members" refers to some or all of the elements of a web, e.g., web **40** in FIG. 1, portions of which serve to define the primary apertures by a continuous network. As can be appreciated from the description of FIG. 1 and the present invention herein, the interconnecting members, e.g., fiber like elements **42, 43, 44, 45**, and **46**, are structurally continuous, with contiguous interconnecting elements blending into one another In mutually adjoining transition portions. Individual interconnecting members can be best described with reference to FIG. 1 as those portions of the web disposed between any two adjacent primary apertures, originating in the first surface and extending into the second surface. On the first surface of the web the interconnecting members collectively form a continuous network, or pattern, the continuous network of interconnecting members defining the primary apertures, and on the second surface of the web interconnecting sidewalls of the interconnecting members collectively form a discontinuous pattern of secondary apertures.

In a three-dimensional, macroscopically-expanded web, the interconnecting members may be described as channel-like. Their two dimensional cross-section may also be described as "U-shaped", as in the aforementioned Radel '314 patent, or "upwardly concave-shaped", as disclosed in U.S. Patent No. 5,514,105, issued on May 7, 1996 to Goodman, Jr., et al. "Upwardly-concave-shaped" as used herein, and as represented in FIG. 1, describes the orientation of the channel-like shape of the interconnecting members with relation to the surfaces of the web, with a base portion **51** generally in the first surface **50**, and the legs, e.g., sidewall portions **53**, of the channel extending from the base portion **51** in the direction of the second surface **55**, with the channel opening being substantially in the second surface **55**. In general, for a plane cutting through the web, e.g., web **40**, orthogonal to the plane, e.g., plane **52**, of the first surface **50** and intersecting any two adjacent primary apertures, e.g., apertures **41**, the resulting cross-section of an interconnecting member disposed therein will exhibit a generally upwardly concave shape that may be substantially U-shaped.

The term "continuous" when used herein to describe the first surface of a macroscopically-expanded, three-dimensional formed film web, refers to the uninterrupted character of the first surface generally in the plane of the first surface. Thus, any point on the first surface can be reached from any other point on the first surface without substantially leaving the first surface. Conversely, as utilized herein, the term "discontinuous" when used to describe the second surface of a three-dimensionally formed film web refers to the interrupted character of the second surface generally in the plane of the second surface. Thus, any point on the second surface cannot necessarily be reached from any other point on the second surface without substantially leaving the second surface in the plane of the second surface.

FIG. 2 shows an enlarged, partially segmented, perspective illustration of a portion of another prior art polymeric microapertured web **110** formed generally in accordance with U.S. Pat. No. 4,629,643, issued to Curro et al. The micro-apertured surface aberrations **120** can be formed by a hydroforming process in which a high-pressure liquid jet is utilized to force the web to conform to a three-dimensional support member. As shown, ruptures which coincide substantially with the maximum amplitude of each micro-apertured surface aberration **120** result in the formation of a volcano-shaped aperture **125** having relatively thin, irregularly shaped petals **126** about its periphery. The relatively thin, petal-shaped edges **128** of the aperture of such a web provide for increased softness impression on the skin of a user when compared, for example, to the web of Ahr '045. It is believed that this softness impression is due to the relative lack of resistance to compression and shear afforded by the surface aberrations having volcano-shaped apertures. For webs of the type depicted in FIG. 2 having both macro-apertures (as shown in the web of FIG. 1) and micro-apertures extending from first surface **50**, the caliper **H** is the dimension from plane **56** of second surface **55** to a plane of a surface corresponding generally to the petal-shaped edges **128** of volcano-shaped apertures **125**.

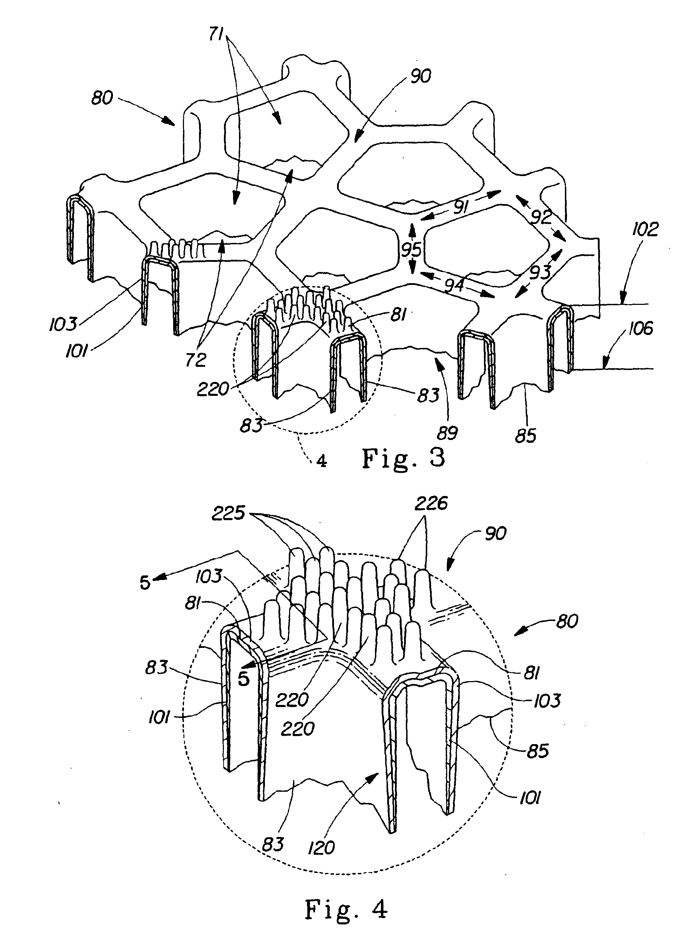

FIG. 3 is an enlarged, partially segmented perspective illustration of a fluid pervious, macroscopically-expanded, three-dimensional polymeric web **80** as taught in U.S. Application No. 10/324,366, filed December 20, 2002, and entitled Polymeric Web Exhibiting a Soft and Silky Tactile Impression. The geometric configuration of the macroscopic surface aberrations, e.g., primary apertures **71**, of the polymeric web can be generally similar to that of the web **40** illustrated in FIG. 1. Primary apertures **71** may be referred to as "apertures" or "macro-apertures" herein, and refer to openings in the web that permit fluid communication between a first surface **90** of web **80** and a second surface **85** of web **80**. The primary apertures **71** of the web shown in FIG. 3 are defined in the plane **102** of first surface **90** by a continuous network of interconnecting members, e.g., members **91, 92, 93, 94,** and **95** interconnected to one another. The shape of primary apertures **71** as projected in the plane of the first surface **90** may be in the shape of polygons, e.g., squares, hexagons, etc., in an ordered or random pattern. In a preferred embodiment primary apertures **71** are in the shape of modified ovals, and in one embodiment primary apertures **71** are in the general shape of a tear drop. Polymer web **80** exhibits a plurality of surface aberrations **220** in the form of hair-like fibrils **225**, described more fully below. For webs of the type depicted in FIG. 3 having both macro-apertures **71** and hair-like fibrils **225** extending from first surface **90**, the caliper **H** is the dimension from plane **106** of second surface **85** to a plane of a surface corresponding generally to the distal ends **226** of hair-like fibrils **225**.

In a three-dimensional, microapertured polymeric web **80**, each interconnecting member comprises a base portion, e.g., base portion **81**, located generally in plane **102**, and each base portion has sidewall portions, e.g., sidewall portions **83** extending from each longitudinal edge thereof. Sidewall portions **83** extend generally in the direction of the second surface **85** of the web **80** and join to sidewalls of adjoining interconnecting members intermediate the first and second surfaces, **90** and **85**, respectively, and terminate substantially concurrently with one another to define secondary apertures, e.g., secondary apertures **72** in the plane **106** of second surface **85**.

FIG. 4 is a further enlarged, partial view of the three-dimensional polymeric web **80** shown in FIG. 3. The three-dimensional polymeric web **80** comprises a polymer film **120**, i.e., the precursor film, which can be a single layer of extruded polymer or a multilayer co-extruded or laminate film comprising two or more layers. As shown in FIG. 4, film **120** is a two-layer laminate comprising a first layer **101** and a second layer **103.** Laminate materials may be co-extruded, as is known in the art for making laminate films, including films comprising skin layers. While it is presently preferred that, as shown in FIG. 4, the polymeric layers, e.g., layers **101** and **103**, terminate substantially concurrently in the plane of the second surface **106** it is not presently believed to be essential that they do so. One or more layers may extend further toward the second surface than the other(s).

FIG. 4 shows a plurality of surface aberrations **220** in the form of hair-like fibrils **225**. The hair-like fibrils are formed as protruded extensions of the polymeric web **80**, generally on the first surface **90** thereof. The number, size, and distribution of hair-like fibrils **225** on polymeric web **80** can be predetermined based on desired skin feel. For applications as a topsheet in disposable absorbent articles, it is preferred that hair-like fibrils **225** protrude only from the base portion **81** in first surface **90** of polymeric web **80**, as shown in FIGS. 3 and 4. Therefore, when web **80** is used as a topsheet in a disposable absorbent article, the web can be oriented such that the hair-like fibrils **225** are skin contacting for superior softness impression, and yet, the hair-like fibrils **225** do not obstruct fluid flow through macro-apertures **71**. Moreover, having hair-like fibrils **225** with closed distal portions **226** results in reduced rewet, i.e., reduced amounts of fluid being re-introduced to the surface of the topsheet after having been first passed through the topsheet to underlying absorbent layers.

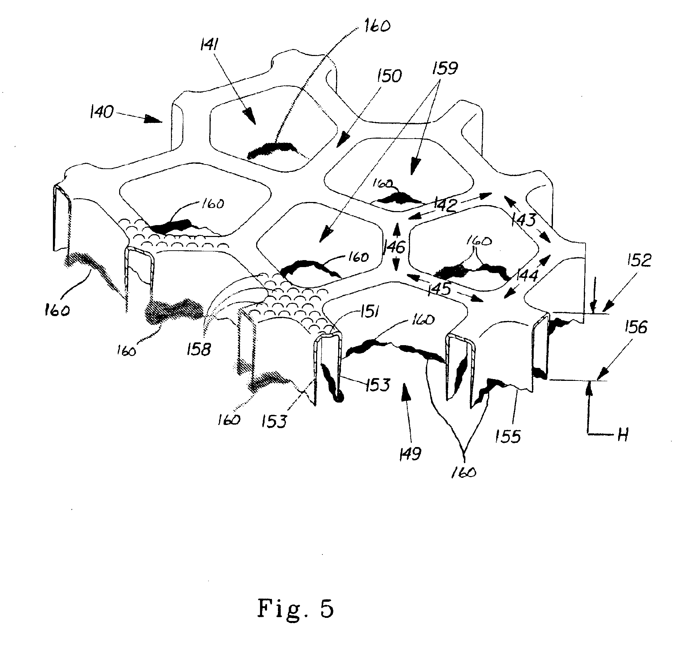

FIG. 5 shows a web of the present invention, the web **140** being in this embodiment in all respects like the web of FIG. 1 but having printed on the second surface **155** thereof a colorant, the colorant in this embodiment being present in the form of ink deposits **160**. Ink deposits **160** can be described as discrete, spaced apart deposits of ink. Colorant can be any of colorants suitable for deposition onto a polymeric film, including water-based inks and dyes, solvent-based inks and dyes, UV-curable inks or dyes, paint, pigment, or liquid colorants such as food coloring. Colorant can be produce a color contrasting with the color of the web **140** and can be primary colors and common colors such as red, green, blue, yellow, pink, purple, orange, or black. If web **140** is dark colored, colorant can be light colors such as light gray, silver, white, or beige. The fluid pervious web **140** exhibits a plurality of macroscopic surface aberrations that can be apertures, such as primary apertures **141.** Primary apertures **141** are formed by a multiplicity of interconnecting members, such as fiber like elements, e.g., **142, 143, 144, 145** and **146**, that are interconnected to one another to define a continuous first surface **150** of the web **140**. Each fiber like element has a base portion, e.g., base portion **151**, located in plane **152** of first surface **150**. Each base portion has a sidewall portion, e.g., sidewall portion **153**, attached to each longitudinal edge thereof. The sidewall portions extend generally in the direction of a discontinuous second surface **155** of web **140**. The intersecting sidewall portions are interconnected to one another intermediate the first and second surfaces of the web, and terminate substantially concurrently with one another in the plane **156** of the second surface **155**. Planes **152** and **156** can be substantially parallel and separated by a distance termed herein as thickness or caliper H. In general, caliper **H** of webs of the present invention useful as topsheets on disposable absorbent articles can be in the range of 0.1 to 2 millimeters, and can range in 0.01 mm increments between these values. Caliper **H** can be from 0.40 to 0.60 millimeters. Caliper **H** can average about 0.44 millimeters. In some embodiments, the base portion **151** may have surface aberrations **158** in accordance with U.S. Patent No. 4,463,045 issued to Ahr et al. on July 31, 1984.

In some embodiments, sidewall portions **153** end substantially uniformly such that the second surface **155** coincides substantially completely with plane **156**. However, in some embodiments, sidewall portions **153** can end in substantially non-uniformly, such as in a somewhat jagged pattern wherein various portions have differing height **H** dimensions. In such an embodiment, plane **156** can be considered located at an average distance **H** determined by second surface **155**. In each embodiment, sidewall portions are considered to extend to a second surface in a second plane of the web.

Ink deposits **160** are shown in FIG. 5 as being predominantly at the distal ends of sidewalls **153**, i.e., substantially only on the second surface **155**. Because sidewalls can be relatively thin, including being thinned from the original thickness of the starting film web material due to the process of making, and because ink can be deposited in droplets having dimensions greater than the sidewall thickness, in some embodiments ink deposits can extend some distance on the sidewall **153** away form the distal end, and yet still be considered substantially only on the second surface **155**. Therefore, in one embodiment, colorants in the form of ink deposits **160** on the second surface **155** can be on the film edge, or primarily on the film edge at the distal ends of sidewall portions **153**. In other embodiments, in addition to being on the film edge at the distal ends of sidewall portions **153**, ink can be on other parts of the sidewall portions **153**, including on the inner or outer surfaces of sidewall portions **153**.

One advantage to having colorant deposited on at least a portion of the second surface of a three-dimensional, formed film web, such as a web illustrated in FIG. 5, is the impression of depth that is created. When used as a topsheet on an absorbent article, a web having colorant deposited at a plane below the top surface provides for a visual impression of depth and richness that is pleasing to consumers. In addition, because the colorant is actually placed at a plane below the top surface, the colorant is, in fact, as far away from the user's skin when the web is used as a topsheet on an absorbent article. This remote placement can be beneficial for certain colorants in which it is undesirable for the colorant to be on or near the top surface of the topsheet.

One advantage to the web of the present invention, and the methods for making as described below, is that colorant, or a combination of colorants can be applied to the web at differing areas of sidewall portions **153**. For example, a colorant can be applied to be limited to being at substantially second surface **155**. Or the colorant, or another colorant having a different color, can be applied midway between first surface **150** and second surface **153**. If more than one color of colorant is used, multiple planes of color can be created to provide novel visual effects to a user viewing the web from the first surface side. When used as a topsheet in an absorbent article, various colorants, colors of colorants, and placement options can be combined to create novel visual shapes, depth effects, color combinations. By way of example, in one embodiment, a first colorant can be applied to the second surface of a central region of a topsheet for a sanitary napkin, and a second colorant having a different color can be applied to the second surface of a side or end region of the topsheet. In another embodiment, a first colorant can be applied to the second surface of a central region of a topsheet for a sanitary napkin in a swirl shape, and a second colorant having a different color can be applied to the mid-sidewall portions in the same region of the topsheet, and in the same swirl pattern to provide for an optical effect. Of course, any combination of colorant, color, deposition location, shape of deposition can be contemplated, each having its own novel visual effect.

Ink deposits **160** on the second surface **155** of web **140** can be applied by a printing process, such as gravure printing and flexographic printing as described below. Other printing processes as are known in the art can be used, each with various advantages and disadvantages. The advantage of printing via a flexographic printing process is that the nip setting at the printing stage can be adjusted and set such that only the second surface **155** of web **140** contacts the inked roll in a highly controllable manner. The result is that ink deposits **160** can be precisely controlled so as to be applied on a surface of web **140** spaced a predetermined distance from first surface **150**. As discussed above, when web **140** is used as a topsheet on a disposable absorbent article, ink deposits **160** on the second surface **155** can render a perception of depth visible from the user-facing side of the disposable absorbent garment. By achieving a perception of depth in this manner, underlying layers such as secondary topsheets having printed signals can be eliminated without loss of the color signal capability. Also, by printing only on the second surface **155** of a formed film, the amount of ink utilized for a commercially viable visual signal can be significantly reduced, as compared to printing a color signal on an underlying layer, such as a secondary topsheet or core layer.

Ink deposits **160** can be closely spaced so as to form a substantially complete coverage of second surface **155**, or they can be spaced relatively far apart. The ink deposits **160** can be limited to substantially only on the very ends of sidewall portions **153**, or the ink deposit **160** can extend a substantial distance up sidewall portion **153** in the direction of first surface **150**. In one embodiment ink deposits **160** are colorfast so that they do not dissolve, degrade, or run when insulted with at least one of water, urine, or menses. In another embodiment, ink deposits **160** can be soluble in at least one of water, urine, or menses, such that upon liquid insult the imprinted color changes or disappears. Such color change can indicate wetness, volume of fluid, position of fluid, and/or type of fluid.

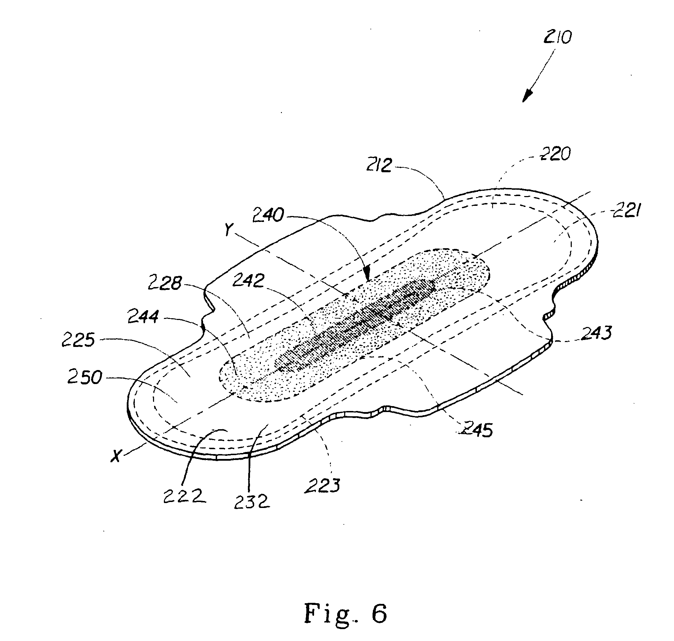

In one embodiment, ink deposits **160** can be printed so as to make a graphic image visible from the body-facing side of a disposable absorbent article. For example, as shown in FIG. 6, the image can be similar to that taught in co-pending, co-owned U.S. Patent Application No. 10/025,059, filed December 19, 2001, entitled Absorbent Article, or co-pending, co-owned U.S. Patent Application No. 10/967,454, filed October 18, 2004, entitled Absorbent Article.

FIG. 6 provides a perspective view of the absorbent article **210**. The absorbent article **210** herein has an upper surface **222**, a lower surface (not seen), and a periphery **212** comprising a topsheet **225** having a bottom surface (not shown) and a viewing surface **232** positioned opposite to the bottom surface. The viewing surface **232** faces upwardly towards the upper surface **222** of the absorbent article **210**. The absorbent article **210** further comprises a backsheet **223** having a garment facing surface (not shown) and a user facing surface positioned oppositely to the garment facing surface, the backsheet **223** being joined to the topsheet **225**, as is common and known in the art. The absorbent article **210** also comprises an absorbent core **220** having a top surface **221** and a bottom surface (not shown) that is positioned opposite to the top surface **221**. The absorbent core **220** is positioned between the topsheet **225** and the backsheet **223**, as is common and known in the art.

The ink deposits **160** can render visible from the viewing surface **232** a graphic, such as graphic **240** shown in FIG. 6. In the embodiment shown in FIG. 6 the absorbent article **210** has at least two portions, i.e., a portion comprising graphic **240** and a portion **250** which does not comprise a graphic **240**. The graphic **240** and the non-graphic portion **250** can be viewable from the viewing surface **232** of the topsheet **225**. The graphic **240** can have at least two shades, a first shade **242** and a second shade **244**. In one embodiment, as is shown in FIG. 6, the first shade **242** can be positioned substantially within the second shade **244**. The second shade **244** can be different, either in lightness, darkness, and/or color, from the first shade **242**. The multi- shades can operate to create an additional perception of depth within the absorbent article by a user looking upon the viewing surface **22** of the topsheet **225**.

While the graphic shown in FIG. 6 is similar to the graphic shown in the aforementioned U.S. Patent Application No. 10/025,059, in the present invention the shape, size, coloration, placement, and intensity of graphic **240** can be varied in ways limited only by the size of the substrate and the printing techniques employed. For example, by use of letter press, lithographic, screen printing, flexographic or gravure printing techniques, virtually any graphic in any color or color combination can be rendered on topsheet **225**. Moreover, by adjusting processing variables such as the nip between rollers in a flexographic process, the amount of ink and the position of ink can be varied to give various impressions of color intensity, brightness/darkness, hue, saturation, and depth perception.

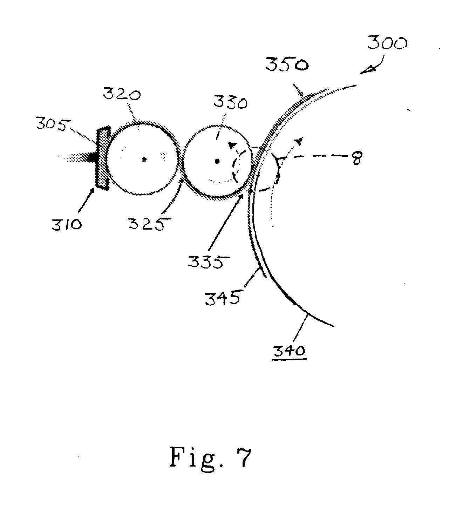

In one embodiment, graphic **240** or other printed image can be imparted to a web, such as topsheet **225** by means of a flexographic printing process, such as that shown schematically in FIG. 7. Flexographic printing process **300** can utilize any of known flexographic printing apparatus and equipment, including processing means known in the art. As shown, ink **305** is supplied in a chamber **310** comprising a doctor blade as is known in the art, and which is in operative relationship with an anilox roller **320** to which ink from the chamber **310** is transferred in a uniform manner as the anilox roller **320** rotates in the direction indicated. Anilox roller **320** is in operative relationship with a plate roller **330** such that at transfer nip **340** ink is transferred from the anilox roller **320** to the plate roller **330**. The plate roller **330** picks up ink from the anilox roller **320** in a pattern corresponding to the pattern desired to be printed on a substrate **345**. The substrate **345** on which the pattern will be printed, such as a web for a topsheet of a disposable absorbent article, such as the sanitary napkin of FIG. 6, is fed onto the central impression drum **340** (in any conventional manner, not shown), which is rotating in the direction shown in FIG. 7.

As substrate **345** enters the printing nip **335** formed by the operational relationship of the surfaces of the plate roller **330** and the central impression roller **340**, the ink **305** on the surface of the plate roller **330** makes contact with, and is transferred to, the substrate 345. Sometimes referred to as an ink impression, the ink transfer pattern corresponds to the graphic **240** that is then visible on printed web **350** and will ultimately be visible from the viewing surface **232** of the topsheet 225 of an absorbent article.

Alternatives to the described flexographic printing process can be implemented. For example, a plurality of printing nips **335** can be arranged, each with its corresponding ink supply and anilox/plate rollers such that multiple patterns and graphics in multiple colors and color intensities can be imparted to substrate **345**. In one embodiment, two such printing nips **335** can be used, each nip delivering color in a registered pattern to render a two-color graphic image to substrate **345**, which image can then be registered so as to be appropriately disposed on the viewing surface **232** of the topsheet **225** of an absorbent article. In some embodiments, when multiple print operations are performed, it can be necessary that the ink from one printing operation dry sufficiently before the next printing operation. Therefore, in one embodiment, between printing operations there can be operatively positioned drying means, such as infra-red heating, UV light curing, forced air drying, and the like, as is known in the art.

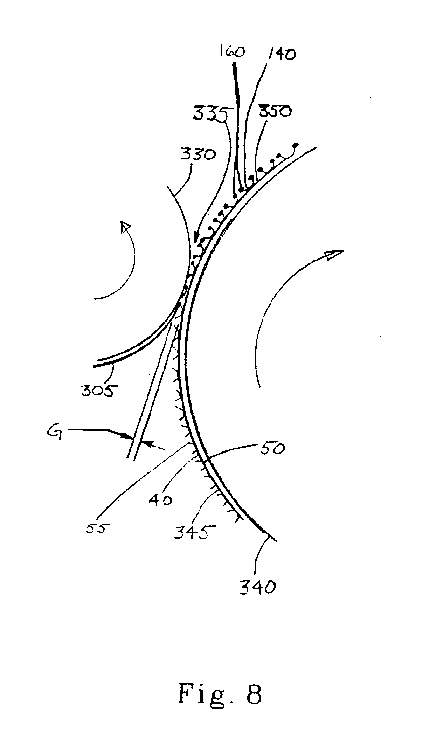

There are many advantages to printing images on three-dimensional, formed film webs by means of the present invention. For example, as shown in FIG. 8, a web, such as web **40** shown in FIG. 1 can be printed in a flexographic printing process with the first surface **50** facing into or on the central impression drum **340** and second surface **55** facing outwardly with respect to the central impression drum **340**. The gap G between plate roller **330** and the central impression roller **340** can be set such that ink transfers only to the second surface **55** of web **40** that, in this embodiment, is the substrate **345** that gets printed to be the printed web **350**. Once printed, the printed web **350** is a web **140** of the present invention as depicted in FIG. 5. Ink has been transferred via the flexographic process to be deposited only on the second surface 155 and, depending on the gap **G**, on portions of side walls **153**.

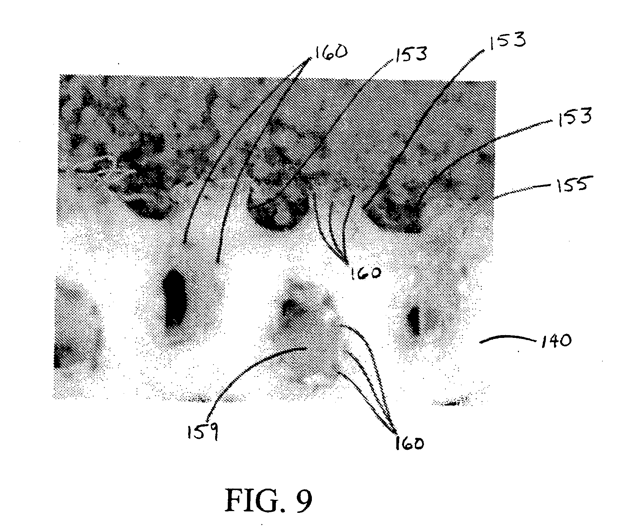

Gap **G** can be adjusted depending on the caliper **H** of the three-dimensional, formed film webs substrate **345**. In one embodiment, gap **G** can be substantially equal to caliper **H** such that only the very ends of sidewall portions **153** in second surface **155** have ink deposited thereon. In another embodiment, gap **G** can be less than caliper **H** such that sidewall portions **153** are compressed through printing nip **335**, resulting in ink being deposited on a portion of the sidewalls **153**. FIG. 9 is a photomicrograph showing about a 3 mm long (horizontally in FIG. 9) section of the second surface **155** of a web **140** of the present invention showing ink deposits **160** on the ends of sidewall portions **153** at substantially the second surface **155**. FIG. 9 shows the underside of a web similar to that shown in FIG. 5, the "underside" being used to denote the side of the web opposite first surface **150**, and including the second surface **155**. In actual use the either side could be the "underside" so the term is used here simply to denote the side printed, which happens to be the underside as oriented in FIG. 5, for example. As configured in FIG. 9, web **140** is folded so as to better show at the top of the FIG., the cone-like structure formed by the sidewall portions **153**. As shown, the ink **305** is printed in a pattern of closely spaced dots on the distal ends of the sidewall portions **153** by a flexographic printing process as described above with respect to FIG. 7. For the three-dimensional, formed film webs substrate **345** printed and depicted in FIG. 9 as web **140**, the ink is a water based ink available as GCOFW7835747 RD 11 from Sun Chemical, applied to a three-dimensional, formed film made by Tredegar Film Products, Haung Pu plant in Guangzhou, GD, China, having a caliper **H** of 0.44mm (when measured under load of 14.7 g/sq cm), in a flexographic PROGLIDE® printing process, made by Mark Andy / Comco, Inc. 910 Lila Ave., Milford, Ohio, in which gap **G** was adjusted to be about 0.01" (0.254mm).

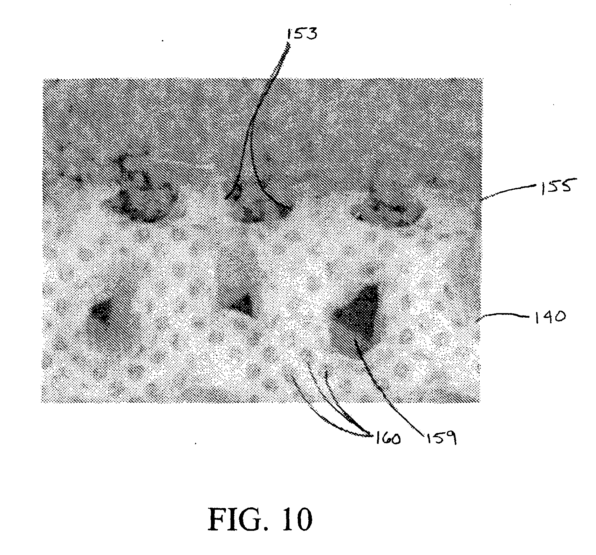

In another embodiment, gap **G** can be adjusted such that at printing nip **335** three-dimensional, formed film webs substrate **345** is significantly compressed such that ink is deposited on the virtually all surfaces of printed web **140** except the first surface, such as first surface **50** of the web shown in FIG. 1. FIG. 10 is a photomicrograph showing about a 3 mm long (horizontally in FIG. 10) section of the second surface **155** of a web of the present invention showing ink deposited on virtually the entire underside of web **140**, the term "underside" being used to denote the side of the web opposite first surface **150**, and including the second surface **155**. In actual use the either side could be the "underside" so the term is used here simply to denote the side printed, which happens to be the underside as oriented in FIG. 5, for example. As configured in FIG. 10, web **140** is folded so as to better show at the top of the FIG. the cone-like structure formed by the sidewall portions **153**. As shown in FIG. 10, the cone-like structures have been somewhat deformed by compression in the printing process, and the ink **305** is printed in a pattern of closely spaced dots over virtually the entire underside of the web by a flexographic printing process as described above with respect to FIG. 7. For the three-dimensional, formed film webs substrate **345** printed and depicted in FIG. 10, the ink is a solvent based ink available as CROFS7711705 DPO-149 made by Sun Chemical, applied to a three-dimensional, formed film made by Tredegar Film Products, Haung Pu plant in Guangzhou, GD, China having a caliper H of 0.44mm (when measured under load of 14.7 g/sq cm) in a flexographic PROGLIDE® printing process made by Mark Andy / Comco, Inc. 910 Lila Ave., Milford, Ohio in which gap **G** was adjusted to be about 0.0" (0.0mm).

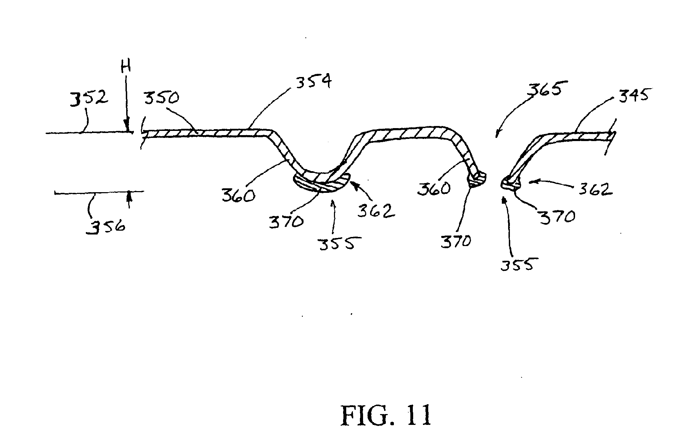

In one embodiment the web of the present invention can be described as a three-dimensional, fluid pervious, web comprising a pattern of protrusions. As shown in FIG. 11, the substrate **345** can comprise a nonwoven web **350** made by known processes including meltblown, spunbond, carding, airlaid, and wetlaid (e.g., paper), and the fibers can be synthetic, bicomponent, cellulosic, nanofibers, microfibers, shaped fibers, surfactant treated fibers, and other fiber types as known in the art, and the web can have sufficient light transmittance such that a printed signal on one side can be seen through the web to the other side. Protrusions **360** can be produced by means known in the art, including hot-pin punching, hot-pin aperturing, embossing, hydroforming, and vacuum forming. Two protrusions **360** are shown in the partial cross sectional view of FIG. 11, one having a closed distal end and one having an open, apertured distal end. Protrusions **360** can extend in sidewall portions to distal ends **362** that collectively define a discontinuous second surface **355** in a second plane **356** of the web. Colorant **370**, such as ink or dye, can be deposited on at least a portion of the distal ends **362** of protrusions **360**, i.e., on the second surface **355** of the web. Protrusions **360** can include or define apertures **365**, the apertures being defined in a first surface **354** of said web in a first plane **352** of the web, and extending in sidewall portions to apertures substantially in the second plane thereof. The distance **H**, which can be considered as the effective thickness of the three-dimensional web **350**, between the first plane **352** and the second plane **356** can be twice the average thickness of the base nonwoven web, or three times, or four times, or at least about five times. A web of this type can be beneficially used in sanitary napkins because a topsheet utilizing such a web does not require an underlying layer to provide a color signal visible to a user from the topsheet surface. Further, by printing on a nonwoven web utilized as a topsheet, the manufacturer can eliminate a layer of material such as a secondary topsheet if the secondary topsheet was otherwise utilized as a printing surface on which to print a color signal visible to a user from the topsheet surface. Further, printing a color signal on the nonwoven web utilized as a topsheet as described herein utilizes less ink or other printed medium to achieve an acceptable level of visual perception by a user. By printing ink, for example, only on the distal portions of, i.e., the tips, of the protrusions of the second surface, a relatively lesser amount of ink can suffice to provide a commercially-acceptable visual signal to a user of a disposable absorbent product.

In one embodiment, therefore, the web of the present invention can be described as a three-dimensional, fluid pervious, polymeric web, the web comprising a pattern of interconnecting members, the interconnecting members defining apertures, the apertures being defined in a first surface of said web in a first plane of said web, and extending in sidewall portions to a second surface in a second plane of the web, and wherein ink is deposited on at least a portion of the second surface of the web. A web of this type, sometimes referred to as a macroscopically-expanded formed film, has beneficial use in sanitary napkins, because a topsheet utilizing such a web does not require an underlying layer to provide a color signal visible to a user from the topsheet surface. Further, by printing on a web utilized as a topsheet, the manufacturer can eliminate a layer of material such as a secondary topsheet if the secondary topsheet was otherwise utilized as a printing surface on which to print a color signal visible to a user from the topsheet surface. Further, printing a color signal on the web utilized as a topsheet as described herein utilizes less ink or other printed medium to achieve an acceptable level of visual perception by a user. By printing ink, for example, only on the tips of the macroscopically-expanded cones of the second surface, a relatively lesser amount of ink can suffice to provide a commercially-acceptable visual signal to a user of a disposable absorbent product.

In another embodiment, lotions, surfactants, creams, and other compositions providing skin health benefits can be applied to the second surface of a web, wherein the description above applies, with, for example, lotion substituted for a colorant such as ink or dye. In one embodiment, for example, a clear or color-tinted lotion of petrolatum can be applied by known processes, such as by sufficiently heating for application by flexo-graphic printing means. Once deposited on the web, the heated petrolatum composition can then be cooled to solidify in place, such as on sidewall portions of the web. In this manner, lotions and lotion compositions can be applied alone, or as the colorant, or with a colorant, or in combination with multiple colorants in multiple locations on the web of the present invention, to provide for skin health benefits. Lotions compositions, for example, can be applied to the second surface of a web utilized as a topsheet, such that during use, due to body heat, wearing motion, or fluid migration, the lotion composition can come into contact with the wearer's skin.

In another embodiment, a nonwoven/formed film laminate can be printed. In one embodiment, a nonwoven web can be joined, such as by adhesive or by thermal bonding to a surface of a formed film, either before or after forming into a three-dimensional, fluid pervious, polymeric web. In this manner, a formed film having a soft, fibrous surface can be achieved.

In another embodiment, webs having tufts formed therein, including laminates of films and nonwovens, can be printed as disclosed herein. In one embodiment, the tufts can be formed by needle punching, or by the rotary methods known as SELF ing, rotary knife aperturing, and the like, as disclosed in co-pending, commonly assigned US Ser. Nos. 11/156,020, filed 17 June 2005; 10/737,306, filed 16 December 2003; 10/737,430, filed 16 December 2003. The method of the present invention can be utilized to deposit a lotion, colorant, or other fluid material on the tips of tufts. In one embodiment, the method of the present invention can be used to print inks or dyes only on the tips of tufts of a tufted nonwoven or laminate web.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A disposable absorbent article comprising a topsheet, said topsheet comprising a three-dimensional, fluid pervious, polymeric web, said web comprising a pattern of interconnecting members, said interconnecting members defining apertures, said apertures being defined in a first surface of said web in a first plane of said web, said first surface being the top surface,and extending in sidewall portions to a second surface in a second plane of said web, **characterized in that** a colorant is deposited on at least a portion of said second surface of said web, said colorant being present as discrete, spaced apart deposits of ink.

2. The disposable absorbent article of Claim 1, wherein said article is selected from the group consisting of a disposable diaper, a urinary incontinence device, a sanitary napkin, and a pantiliner.

3. The disposable absorbent article of Claim 2, wherein said article is a sanitary napkin, and said ink is applied by printing on said second surface.

4. The disposable absorbant article of anyone of the preceding claims, wherein said sidewall portions extend to distal ends, said distal ends collectively defining a discontinuous second surface in said second plane of said web, said colorant being deposited on said distal ends

5. The disposable absorbant article of any one of the preceding claims , wherein said colorant is selected from the group consisting of water-based inks and solvent-based inks.

6. The disposable absorbant article of any one of the preceding claims wherein said colorant is deposited on at least a portion of said sidewall portions.

## Patentansprüche

1. Wegwerf-Absorptionsartikel, der eine obere Lage umfasst, wobei die obere Lage eine dreidimensionale, fluiddurchlässige, polymere Bahn umfasst, wobei diese Bahn ein Muster aus miteinander verbundenen Elementen umfasst, wobei die miteinander verbundenen Elemente Öffnungen definieren, wobei die Öffnungen in einer ersten Oberfläche der Bahn in einer ersten Ebene der Bahn definiert sind, wobei die erste Oberfläche die Oberseite ist, und sich in Seitenwandabschnitten zu einer zweiten Oberfläche in einer zweiten Ebene der Bahn erstrecken, **dadurch gekennzeichnet, dass** ein Farbmittel auf mindestens einem Abschnitt der zweiten Oberfläche der Bahn aufgebracht ist, wobei das Farbmittel als diskrete, voneinander beabstandete Tinteabscheidungen vorliegt.

2. Wegwerf-Absorptionsartikel nach Anspruch 1, wobei der Artikel ausgewählt ist aus der Gruppe bestehend aus einer Wegwerfwindel, einer Urininkontinenzvorrichtung, einer Damenbinde und einer Slipeinlage.

3. Wegwerf-Absorptionsartikel nach Anspruch 2, wobei der Artikel eine Damenbinde ist und die Tinte durch Bedrucken der zweiten Oberfläche aufgebracht wurde.

4. Wegwerf-Absorptionsartikel nach einem der vorstehenden Ansprüche, wobei die Seitenwandabschnitte sich zu distalen Enden erstrecken, wobei die distalen Enden gemeinsam eine diskontinuierliche zweite Oberfläche in der zweiten Ebene der Bahn definieren, wobei das Farbmittel auf den distalen Enden aufgebracht wurde.

5. Wegwerf-Absorptionsartikel nach einem der vorstehenden Ansprüche, wobei das Farbmittel ausgewählt ist aus der Gruppe bestehend aus wasserbasierten Tinten und lösungsmittelbasierten Tinten.

6. Wegwerf-Absorptionsartikel nach einem der vorstehenden Ansprüche, wobei das Farbmittel auf mindestens einem Abschnitt der Seitenwandabschnitte aufgebracht wurde.

## Revendications

1. Article absorbant jetable comprenant une feuille de dessus, ladite feuille de dessus comprenant une nappe polymère tridimensionnelle, perméable aux fluides, ladite nappe comprenant un motif de membres d'interconnexion, lesdits membres d'interconnexion définissant des ouvertures, lesdites ouvertures étant définies dans une première surface de ladite nappe dans un premier plan de ladite nappe, ladite première surface étant la surface supérieure, et s'étendant dans des parties de paroi latérale vers une deuxième surface dans un deuxième plan de ladite nappe, **caractérisé en ce qu'**un colorant est déposé sur au moins une partie de ladite deuxième surface de ladite nappe, ledit colorant étant présent en tant que dépôts d'encre distincts, espacés.

2. Article absorbant jetable selon la revendication 1, où ledit article est choisi dans le groupe constitué d'une couche jetable, un dispositif pour l'incontinence urinaire, une serviette hygiénique, et un protège-slip.

3. Article absorbant jetable selon la revendication 2, où ledit article est une serviette hygiénique, et ladite encre est appliquée par impression sur ladite deuxième surface.

4. Article absorbant jetable selon l'une quelconque des revendications précédentes, dans lequel lesdites parties de paroi latérale s'étendent jusqu'aux extrémités distales, lesdites extrémités distales définissant collectivement une deuxième surface discontinue dans ledit deuxième plan de ladite nappe, ledit colorant étant déposé sur lesdites extrémités distales.

5. Article absorbant jetable selon l'une quelconque des revendications précédentes, dans lequel ledit colorant est choisi dans le groupe constitué d'encres à base d'eau et d'encres à base de solvant.

6. Article absorbant jetable selon l'une quelconque des revendications précédentes, dans lequel ledit colorant est déposé sur au moins une partie desdites parties de paroi latérale.
